(19) Europäisches Patentamt
European Patent Office
Office européen des brevets

(11) **EP 4 738 273 A1**

(12) **EUROPEAN PATENT APPLICATION**

(43) Date of publication:
**06.05.2026 Bulletin 2026/19**

(21) Application number: **24210148.3**

(22) Date of filing: **31.10.2024**

(51) International Patent Classification (IPC):
*G06V 10/22* (2022.01)    *G06V 10/25* (2022.01)
*G06V 10/764* (2022.01)    *G06V 10/94* (2022.01)
*G06V 30/14* (2022.01)    *G06V 30/19* (2022.01)
*G06F 3/048* (2013.01)    *G16H 30/20* (2018.01)
*G16H 30/40* (2018.01)

(52) Cooperative Patent Classification (CPC):
**G06F 3/048; G06V 10/22; G06V 10/235;
G06V 10/25; G06V 10/764; G06V 10/945;
G16H 30/20; G16H 30/40; G16H 50/20;**
G06V 2201/02; G06V 2201/03

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventor: **GOTMAN, Shlomo
Eindhoven (NL)**

(74) Representative: **Philips Intellectual Property &
Standards
High Tech Campus 52
5656 AG Eindhoven (NL)**

(54) **AUTOMATIC SYNCHRONISATION OF TWO INDEPENDENT IMAGE PROCESSING APPLICATIONS**

(57) A computer-implemented method of image viewing and/or processing within an image viewing and/or processing environment, comprising the following steps:
- obtaining screen capture content of at least part of an image display of the first processing application, wherein the image display is based on image data at least partially loaded into the first processing application;
- automatically attempting to retrieve window characteristic information from the screen capture content based on at least one screen location of window characteristic information from the library of screen locations;
- classifying the screen capture content:
- as recognizable if window characteristic information can be retrieved;
- as non-recognizable if the library of screen locations currently does not contain any screen locations and/or if no window characteristic information can be retrieved;
- in case the screen capture content is classified as recognizable:

- providing the window characteristic information to the second processing application;
- synchronizing the window of the second processing application based on the received window characteristic information of the first processing application;
in case the screen capture content is classified as non-recognizable:

- performing a learning process to determine at least one screen location of window characteristic information on the image display based on changes happening over time on the image display and/or based on user input and storing the at least one screen location of window characteristic information in the library of screen locations.

FIG.1

**Description**

FIELD OF THE INVENTION

The invention relates to a computer-implemented method performed within an image viewing and/or processing environment, a corresponding computer program, and a corresponding image visualisation and/or processing computing system.

BACKGROUND OF THE INVENTION

Clinical diagnostic reading of medical images is typically performed by a user on a visualization system such as a PACS (Picture Archiving and Communication System) or a similar system (for example an advanced visualization workstation). While providing basic common viewing capabilities, general visualization systems, such as PACS, often lack the ability for advanced visualization and processing techniques. In addition, they may not be able to leverage proprietary, modality-vendor dependent information stored with the images, or proprietary image formats, like computational tomography (CT) spectral based images (SBI). Corresponding functionalities and/or further applications may, however, be provided by different, specific programs, e.g. programs provided by different vendors.

To provide these additional capabilities, a possible solution is to provide an add-on application, e.g. a processing application, for example in the form of a software application that is running in the PACS environment (e.g., on a PACS workstation), as a client in a client-server architecture, or as a web page. These processing application may for example provide advanced visualization capabilities and/or computer aided diagnostic capabilities that go beyond the general visualization system.

Accordingly, there may be two different applications running in the same environment but as two different applications. However, the two different applications may in many cases not be capable of sharing any information. This is particularly technically problematic in case the two processing applications are from two different vendors. It would, however, be desirable to enable such sharing of information in particular information on the characteristics of a window in which image data is displayed by the two different processing applications.

For example, two different image processing applications from two different vendors run on the same workstation, but do not share any window characteristic information and are thus not synchronized. For instance, the two applications can display different images from the same series, using different zoom/pan, different windowing, etc. This means that if the user wants to compare the images, there is a need to do this separately on each application, jumping from application to another which means significant extra burden to the reading workflow.

For example, sharing window characteristic information between processing applications may allow a user to experience an integrated environment where it easier to perform technical tasks. This could help the user, such as a radiologist, to save time and improve workflow efficiency while performing these tasks.

One solution could be to provide a non-standard interface, such as an application programming interface (API) or command Unified Resource Locator (URL), to provide synchronization of windows between two processing applications. However, since there is no widely accepted standard for such interfaces, i.e. the interface is non-standard, this typically requires integration of two or more processing applications with each other. One processing application may for example be general processing application provided in a PACS environment and the other application may be an advanced visualization processing application provided by a different vendor.

Integration, of such processing applications from different vendors may be technically problematic, or at least takes significant time to adapt one or both processing applications.

This may be referred to as "tight integration" between applications. However, this approach is often not possible or technically very difficult to implement. It may take a lot of development time and typically requires adaptations of the integration each time any of the two applications is updated to a new version.

Since these interfaces are not standard, this requires integration of the processing application with each PACS vendor separately. In addition, this method is used for one-time invocation of a 3rd party (non-PACS) application, rather than for real-time synchronization.

Another approach could be to universally use only one uniform standard for all the applications, such as CCOW (Clinical Context Object Workgroup standard). However, a uniform standard that is used by all applications has not been established successfully so far and may be difficult or even impossible to establish in the future for various reasons such as technical reasons.

It would therefore be desirable to find a more simple and practicable solution for the above-mentioned problems or at least find an alternative. It is desirable to enable a functional and easier to implement solution for synchronization of windows of different image processing applications.

The present invention allows an automatic instantaneous synchronization between two applications without a need to integrate different processing applications by software integration, nor by compliance with some possible future interface standard (not currently available or industry-widely accepted).

OBJECT OF THE INVENTION

It is, therefore, an object of the invention to provide a method to better address the above-mentioned problems. In particular, an object of the invention is to enable an easier to implement and/or to use solution for synchronizing windows of two different processing applications, particularly in medical image reading environments.

SUMMARY OF THE INVENTION

To better address this object, a method according to claim 1, a computer program according to claim 13, and a system according to claim 14 is provided.

Advantageous embodiments are set out in the dependent claims. Any features, advantages or alternative embodiments described herein in relation to the claimed method are also applicable to the other claim categories and vice versa.

According to a first aspect of the invention, a computer-implemented method of image viewing and/or processing within an image viewing and/or processing environment is provided.

The environment comprises at least a first processing application being configured to receive, process and display image data in a window and a second processing application being configured to receive, process and display image data in a window.

In particular, the image data in the first processing application is the same as the image data in the second processing application. In other words, the first processing application has access to image data to which the second processing application also has access. However, the processing performed by the first processing application and displayed within its respective window may be different from the processing performed by the second processing application. In other words, the same image data may be processed however in a different way, which may result in a different displaying (because of the different processing) of image data in the respective windows of the first- and second processing application. The idea is to synchronize the respective windows of the first- and second processing applications.

The environment comprises a window characteristic recognition program having access to a library of screen locations of window characteristic information and comprises or has access to a window characteristic recognition algorithm. The window characteristic algorithm is configured to recognize window characteristic information at a particular screen location.

The window characteristic information is information suitable to identify the characteristics of a window in which image data is displayed.

Such window characteristic information may for example be any context information that provides information on the characteristics of a window and the image data displayed therein. For example, such window characteristic information may be an image number, image location and/or slice number, slice location that provides context information on the characteristic of a window in the first application.

In another example, such window characteristic information is at least one from the list of: an image number, an image location, an image description, a slice number, a slice location, a slice description, a slice thickness, a series number, a series description, an accession number, a zoom factor, a window centre value, a window width value, a slice number in a series.

The method comprises the step of obtaining, by the window characteristic recognition program, screen capture content of at least part of an image display of the first processing application, wherein the image display is based on image data at least partially loaded into the first processing application.

As discussed above, preferably, the same image data is at least partially loaded into the second processing application.

The method comprises the step of automatically attempting to retrieve window characteristic information, by the window characteristic recognition program, from the screen capture content based on at least one screen location of window characteristic information from the library of screen locations.

In other words, the window characteristic recognition program automatically identifies locations, for example rectangular areas, on the screen where the window characteristic information is displayed.

In a preference, initial manual presets of screen locations with window characteristic information may be used. Such initial manual presets may be used later by a window characteristic recognition algorithm to extract the window characteristic information.

The method comprises classifying the screen capture content, by applying the window characteristic recognition algorithm.

The screen capture content is classified as recognizable if window characteristic information can be retrieved.

The screen capture content is classified as non-recognizable if the library of screen locations currently does not contain any screen locations and/or if no window characteristic information can be retrieved.

In case the screen capture content is classified as recognizable, then the method comprises the step of providing the window characteristic information to the second processing application and synchronizing the window of the second processing application based on the received window characteristic information of the first processing application.

In case the screen capture content is classified as non-recognizable, then the method comprises the step of performing a learning process, by the window characteristic recognition program, to determine at least one screen location of window characteristic information on the image display based on changes happening over time on the image display and/or based on user input and storing the at least one screen location of window characteristic information in the library of screen locations.

Advantageously, the method according to the invention allows sharing of windows characteristic information between two different image processing applications without requiring software integration between the two image processing applications. Additionally, no compliance with some kind of uniform standard is required.

In particular, window characteristic information that includes vendor specific proprietary information, may be identified based on what is displayed, i.e. by screen capturing of the image display of a user interface.

Thus, the computer implemented method may in particular be a method for providing window characteristic information from a first application to another application and vice versa.

The image viewing and/or processing environment comprises in particular an image archiving and communication system such as a PACS (Picture Archiving and Communication System).

The image viewing and/or processing environment may be based on digital computing devices and/or digital networks. The image viewing and/or processing environment may comprise or have access to at least one data storage of image data. The image viewing and/or processing environment may comprise the library of screen locations of window characteristic information.

Alternatively, the environment may have access to the library of screen locations of window characteristic information. The library is preferably a digital library. The library may be stored on a digital storage medium. The image viewing and/or processing environment may comprise at least one server that comprises or is connected to the at least one data storage of image data and/or to the library of screen locations of window characteristic information. The image viewing and/or processing environment may be connected to other systems, such as to a Radiology Information System (RIS) or an advanced visualization workspace (AVW). For example, the image viewing and/or processing environment may be part of or accessible via a workstation. The environment comprises at least a first processing application and a second processing application. The term processing application is to be interpreted broadly. It generally describes a computer program designed to process image data, in particular medical image data, in some way. Processing image data, may for example, comprise one or more of the following: retrieving the image data from a data storage, providing the image data for display on a screen, providing different image views of the image data, providing a digital user interface with the image data for, providing measurements of the image data and/or measurement tools for the image data, making calculations based on the image data, transforming the image data in some way, segmenting objects of interest, fusing image data, assisting in computer aided diagnosis based on the image data and/or performing quantitative analysis on the image data.

The first processing application is configured to at least partially display image data. For example, the first processing application may display a part of an organ that is imaged with the image data. For example, the first processing application may display a slice of three-dimensional image data. Optionally, the second process-

sing application is also configured to at least partially display the same image data.

Hence, in particular, both, the first and the second processing application may be configured to at least partially display the image data. The image data may, in particular be or comprise medical image data. Generally, whenever image data or images are mentioned within the context of this invention, the image data may in particular be medical image data and/or the image may in particular be a medical image. For example, the medical image data may comprise computed tomography image data, magnetic resonance image data, digital X-ray radiogrammetry, and/or AMI (Advanced Molecular Imaging).

Typically, processing applications display information about the image data on the user interface or display that is used by a user. Information about the image data is denoted window characteristic information in the context of this information. In the context of this invention, in particular the first processing application displays window characteristic information. However, it is foreseen that the second processing application may also display window characteristic information.

Window characteristic information is generally any information that is suitable to identify characteristics of a window that comprises the image data for displaying.

In particular, the window characteristic information may comprise a number or name connected with the image data. For example, the window characteristic information may comprise any one or several of an image number, a patient number, a patient name.

Window characteristic information may also be denoted as "context information", "context" or "screen context" that provides context on the characteristic of a window and the image data that is displayed therein.

As part of the method according to the invention, the window characteristic recognition program obtains screen capture content of at least part of an image display of the first processing application. Preferably, the window characteristic recognition program also obtains screen capture content of at least part of an image display of the second processing application.

For example, screen capture content may be a screenshot or part of a screenshot. For example, in some embodiments, only the upper part of the image display may be used. For example, the part of the image display to be used may be based on screen locations of the library of screen locations. The image display is based on image data at least partially loaded into the first processing application and the second processing application.

For example, the image display may comprise a user interface showing a representation of the image data or of part of the image data and, additionally, at least some kind of window characteristic information. The screen capture content may be taken directly by the window characteristic recognition program, for example by making a screen capture of the currently displayed screen and/or the currently displayed user interface of the first processing application.

Alternatively, the window characteristic recognition program may receive the screen capture content. The screen capture content may be provided to the window characteristic recognition program.

For example, another program may take the screen capture content and provide and/or send it to the window characteristic recognition program.

Preferably, the step of obtaining the screen capture content may be initiated based on a user input. Optionally there may be an additional step, in particular before the step of obtaining the screen capture content, of receiving user input to initiate the second processing application to analyse the same image data currently running with the first processing application.

Hence, for example, a user may be looking at image data of a patient with the first processing application and simultaneously want a different analysis of the image data that cannot be provided by the processing application. In this case, the user may want to use a different and/or more powerful processing application that is synchronized and displayed simultaneously with the first processing application.

In the context of this invention the second processing application may be configured to provide different, additional and/or more processing and/or viewing tools than the first processing application in the same view. Typically, if the two processing applications are not integrated with each other, according to the state of the art, the user would have to manually adjust the windows of the first processing application and the second processing application to allow a proper simultaneous and synchronized view. With every manipulation, or change, in one of the processing applications, the windows need to be manually adjusted to continue a technically meaningful simultaneous and synchronized view.

However, the method according to the invention provides a way of automatically determining how the window in the second processing application must be synchronized based on what is currently displayed by the first processing application without needing a specific software integration with the first processing application. The same may apply vice-versa. For example, the method according to the invention provides a way of automatically determining how the window in the first processing application must be synchronized based on what is currently displayed by the second processing application without needing a specific software integration with the first processing application.

In another example, an input option may be offered to the user, such as a hotkey, the activation of which by the user will automatically start the method. For example, the user may press a button "start synchronization of windows displaying same image data but each window representing a different processing application" to initiate the method.

The window characteristic recognition program automatically attempts to retrieve window characteristic information from the screen capture content by applying the window characteristic recognition algorithm. This attempt is based on at least one screen location of window characteristic information from the library of screen locations.

For example, the window characteristic recognition algorithm may be applied to a part of the screen capture content that is defined by the at least one screen location. The window characteristic recognition algorithm may be a text recognition algorithm. The window characteristic recognition algorithm may be an optical character recognition (OCR) algorithm and/or an algorithm comprising a neural network trained to recognize text and/or numbers. The window characteristic recognition algorithm itself may be a recognition algorithm known in the state of the art. For example, the object character recognition (OCR) software "PaddleOCR" may be used.

It is possible that the library of screen locations does initially not contain any screen location. In this case it may be automatically determined that window characteristic information cannot be retrieved at a stored screen location. Optionally, the library of screen locations may comprise exactly one location. The number of screen locations may vary, depending on how often the method according to the invention has been applied, in particular depending on how often the learning process has been applied. The library of screen locations may comprise multiple screen locations. For example, the library may comprise multiple screen locations for multiple types of window characteristic information, in particular one screen location for one type of window characteristic information. Each screen location may be mapped to a particular type of window characteristic information.

Mapping the screen locations to a type of window characteristic information may allow to easily determine what kind of window characteristic information has been retrieved at a particular screen location.

Optionally, the library may only comprise screen locations for one first processing application. Optionally, the library may comprise screen locations for multiple first processing applications. Hence, the library may enable to retrieve window characteristic information from different (first) processing applications.

Optionally, the library may also comprise screen locations for one second processing application. Optionally, the library may comprise screen locations for multiple second processing applications. Hence, the library may enable to retrieve window characteristic information from different (second) processing applications.

The step of classifying may be performed by the window characteristic recognition program. Alternatively, the classification may be performed by an additional classification program.

The screen capture content is classified as recognizable if window characteristic information can be retrieved.

The second processing application has access to the same data as the first processing application. It may be accessed through a different data storage. If the window characteristic information cannot be identified in the data storage, that may mean, that the window characteristic information is wrong, or, in particular when the second processing application accesses different image data than the first processing application, then the windows displaying such image data may not need to be synchronized.

Optionally, in this scenario additional steps may be taken to determine what to do. Alternatively, the window characteristic recognition program may simply do nothing in this case or wait for another attempt to retrieve window characteristic information. Alternatively, the window characteristic recognition program may load the same image data as for the first processing algorithm into the second processing algorithm. Alternatively, the window characteristic program may classify the screen capture content as non-recognizable.

The screen capture content is classified as non-recognizable if the library of screen locations currently does not contain any screen locations and/or if no window characteristic information can be retrieved.

In this case the window characteristic recognition program performs the learning process.

Advantageously, a relatively simple way of initiating the window characteristic recognition program with a new or updated first processing application may thus be provided automatically when the window characteristic information may not yet be retrieved. The screen location of window characteristic information as determined via the learning process is then stored in the library of screen locations.

Hence, the stored screen location for the window characteristic information can be used for future automatic retrieving of window characteristic information. Optionally, the learning process may comprise repeating same or similar steps of the learning process a plurality of times. For example, changes happening on the image display may be tracked until a plurality of changes has been tracked. For example, user input may be recognized a plurality of times. Repeating the steps of the learning process may enable a more reliable determination of screen locations.

If the screen capture content is classified as recognizable, the window characteristic information is provided to the second processing application. This is followed by synchronizing the window of the second processing application based on the received window characteristic information of the first processing application.

In particular the learning process has been applied at least once. Advantageously, the second processing application may thus receive the window characteristic information and synchronizes the window displaying image data of the second processing application with the window displaying image data of the first processing application without needing a direct software integration between the two processing applications and without requiring a user to explicitly make manipulations in the second processing application to make it in sync with the first processing application.

Optionally an additional step may be provided: based on the window characteristic information, at least partially loading the same image data from the data storage into the second processing application. The image data may be displayed in a synchronized manner in a user interface of the second processing application. Advantageously, the user may thus directly continue working (e.g. comparing windows simultaneously) on the image data with the second processing application.

According to an embodiment, the step of obtaining also comprises a step of obtaining screen capture content of at least part of an image display of the second processing application. In other words, the obtained screen capture is from an image display of both the first and second processing applications. In case the screen capture content from the first processing application is classified as recognizable then the mechanism as described above applies. However, at the same time, in case the screen capture content from the second processing application is classified as recognizable, then the method comprises the step of providing the window characteristic information to the first processing application and synchronizing the window of the first processing application based on the received window characteristic information of the second processing application. Similarly, in case the screen capture content (based on the second processing algorithm) is classified as non-recognizable, then the step of performing the learning process comprises the step of determining at least one screen location of window characteristic information on the image display of the second processing application based on changes happening over time on the image display of the second processing application and/or based on user input and storing the at least one screen location of window characteristic information in the library of screen locations. This way, synchronization may be two-way. In other words, it is possible to provide the window characteristic information from a screen capture in the first processing application to the second processing application and vice versa. It is then also possible to synchronize the window of the second processing application based on the received window characteristic information of the first processing application and vice versa, i.e. synchronize the window of the first processing application based on the received window characteristic information of the second processing application.

This allows to show the same image data processed by two different image processing applications in a synchronized manner.

According to an embodiment, the step of synchronizing the window of the second processing application comprises a step of synchronizing by adjusting a zoom factor and/or panning setting for the image data within the window resulting in a similar field of view for the window of the first processing application and the window of the second processing application.

According to an embodiment, the step of synchronizing the window of the second processing application comprises a step of synchronizing by windowing the window, resulting in a similar window of a grey-level mapping.

According to an embodiment, the method further comprises a step of displaying the window of the first processing application and the window of the second processing application simultaneously.

According to an embodiment, the learning process is performed while the first processing application is running. In particular, the learning process may be performed while a user is working with the first processing application.

Alternatively, the learning process is performed while the second processing application is running. In particular, the learning process may be performed while a user is working with the second processing application.

According to an embodiment, the step of performing the learning process further comprises a step of tracking changes on the image display that are due to user input and/or registering user input.

In particular, the user input may be user input that is input by the user while the user is working with the first processing application or the second processing application. For example, a user switching between using the first processing application and the second processing application and/or manipulating a window in either of the first or second processing applications and/or selecting image data may be tracked to determine the window characteristic information.

According to an embodiment the step of performing the learning process comprises tracking a user input concerning a selection of image data and retrieving window characteristic information from the selected image data. The step of performing the learning processes also comprises a step of searching for the window characteristic information on the screen capture content via the window characteristic recognition algorithm to locate the screen location of the corresponding window characteristic information on the screen capture content.

Tracking the user's selection of image data and retrieving window characteristic information directly form the selected image data may allow to determine with good certainty what kind of window characteristic information and/or what specific window characteristic information belongs to the image data.

For example, the window characteristic information may be extracted from the metadata of the image data and/or from the file name of the image data.

For example, at the beginning, when automatically retrieving the window characteristic information does not work yet, in particular due to missing screen locations, the user may still have to manually select the same image data for the second processing application. Preferably, the same image data is loaded into the second processing algorithm automatically.

Tracking what the user selects and comparing it to the screen capture content from the first processing application, may enable a particularly reliable and quick way of learning the screen locations.

The steps of the learning process may be repeated a plurality of times.

For example, the steps may be repeated 2 to 20 times, more preferably 2 to 10 times, most preferably 2 to 5 times.

Advantageously, via this embodiment, the learning can be done during normal work of a user, in particular without requiring the user to perform or initiate some specific calibration procedure.

According to an embodiment the step of obtaining screen capture content comprises obtaining the screen capture content multiple times and/or continuously. The step of performing the learning process comprises a step of analysing one or multiple sections of the screen capture content that are changing over time and determining that or whether the at least one screen location of window characteristic information is within a changing section of the screen capture content.

There may be a plurality of changing sections, meaning that there are multiple screen locations of window characteristic information, in particular different types of window characteristic information.

For example, a background process program, e.g. a daemon, may be used to periodically analyse changing field and/or poll screen texts. Text fields that stay fixed may be ignored. The idea is, that while a user is reviewing image data in the first processing application, the corresponding different window characteristic information may change with each different set of image data, while the same type of window characteristic information may be displayed at the same screen location each time.

For example, fixed fields with non-changing text may be titles, menus, or hospital name (assuming, the data were taken in the same hospital) etc.

For example, changing window characteristic information may be image number and/or image location that is representative for a certain window characteristic.

Advantageously, via this embodiment, the learning can be done during normal work of a user, in particular without requiring the user to perform, actively take part in, or initiate some specific calibration procedure.

According to an embodiment, the step of performing the learning process comprises a step of searching for the window characteristic information via the window characteristic recognition algorithm in the one or multiple changing sections of the screen capture content.

In particular, the two previously described embodiments may be combined. Accordingly, after tracking the user input concerning a selection of image data and retrieving the window characteristic information from the selected image data, the window characteristic information may be searched for in one or multiple changing sections of the screen capture content. Advantageously, the area that needs to be searched can be more limited, which may lead to a quicker and more reliable process. Furthermore, the specific type of window characteristic information may be connected to the screen location more easily.

According to an embodiment, the step of performing the learning process comprises a step of providing a user interface that allows a user an option to select window characteristic information on the image display of the first processing application to input at least one window characteristic information selection and a step of receiving the window characteristic information selection by the window characteristic recognition program. The step of performing the learning process also comprises a step of determining by the window characteristic recognition program and based on the window characteristic information selection, the at least one screen location of window characteristic information.

The selection may comprise selecting a screen area where the window characteristic information is located. Hence, the window characteristic recognition program may be configured to provides a user interface that allows a user the option to select window characteristic information on the image display of the first processing application to input at least one window characteristic information selection, at least during the learning process.

For example, this option may be offered as an alternative to the previously described, fully automatic embodiments. For example, if a user wants to speed up the learning process, this option may be advantageous. Thus, even during the learning process, the user workflow may be enhanced according to this embodiment. For example, a selection feature may be provided to the user to enable the user to mark the screen location. For example, the user may be given the option to draw a geometric figure, such as a rectangle. The corresponding user input may then be received and used to determine the screen location. For example, the user may be prompted to mark, for example draw a rectangle around a specific type of window characteristic information, such as an image number or image location.

According to an embodiment, the learning process comprises searching for the window characteristic information via the window characteristic recognition algorithm in a user selected screen area.

According to an embodiment, the window characteristic information comprises at least one or more from the following list: an image number, an image location, an image description, a slice number slice location, a slice description, a series label, a series description.

According to an embodiment, the image data comprises medical image data. In particular the image data comprises at least one or more from the following list: computed tomography image data, photon counting image data, spectral computed tomography image data, magnetic resonance image data, nuclear imaging data, positron emission tomography data, single photon emission computed tomography imaging data, ultrasound image data, digital pathology data and/or digital X-ray radiogrammetry data.

According to an embodiment, the method comprises a step of storing the at least one screen location of window characteristic information in the library of screen locations during the step of performing the learning process and a step of applying again the previous steps starting with the step of obtaining screen capture content or starting with the step of attempting to retrieve window characteristic information.

Thus, advantageously, different first processing applications may be distinguished allowing to reliably draw window characteristic information from different processing applications. The method may comprise the step of the window characteristic recognition program automatically attempting to retrieve the processing application identification and, based on the application identification recognize the first processing application.

According to an embodiment, after storing the at least one screen location of window characteristic information in the library of screen locations during the learning process, the method is applied again starting with the step of obtaining screen capture content or starting with the step of attempting to retrieve window characteristic information. Hence, the learned screen locations may advantageously be used for future synchronization, in particular immediately after the learning process, either on the existing screen capture content or on following screen capture content.

According to a second aspect of the invention, a computer program is provided that comprises instructions which, when the program is executed by a computer, cause the computer to carry out the steps of the method according to any one of the above embodiments or combinations thereof.

The instructions may comprise any of the method steps and their variants as described herein. The computer program may be stored on a data storage medium, in particular a non-volatile data storage medium. The storage medium may be any computer-readable storage medium. For example, the storage medium may be a solid-state storage medium or an optical storage medium. The storage medium may be a hard-drive, a solid-state-disk, a compact disk, a DVD, a flash storage an online server etc.

According to a third aspect of the invention, an image visualisation and/or processing computing system is provided that comprises a processing unit or processor adapted to perform the steps of the method according to any of the above embodiments or combinations thereof.

In particular, the image visualisation and/or processing computing system has stored thereon a computer program as described herein and a first processing application and a second processing application, at least the first processing application, in particular both processing applications, being configured to at least partially display image data.

According to an embodiment, the image visualization and/or processing computing system comprises a data storage that comprises a library of screen locations.

According to an embodiment, the image visualization and/or processing computing system comprises a data storage, in particular non-transient data storage, with a library of screen locations, in particular a library of screen locations of window characteristic information. Alternatively or additionally, the system may have access to a data storage with a library of screen locations of window characteristic information. The system may comprise at least one data storage of image and/or have access to at least one data storage of image data.

The features and advantages of different embodiments can be combined.

BRIEF DESCRIPTION OF THE DRAWINGS

The invention shall now be illustrated with reference to the attached drawings, in which:

Fig. 1 shows an example of screen capture content of an image display of a first processing application according to an embodiment of the invention;
Fig. 2 shows a schematic describing an image visualisation and/or processing computing system according to an embodiment of the invention;
Fig. 3 shows a flow diagram of a method according to an embodiment of the invention;
Fig. 4 shows a flow diagram of a learning process of a method according to an embodiment of the invention;
Fig. 5 shows a flow diagram of a learning process of a method according to another embodiment of the invention;
Fig. 6 shows a flow diagram of a learning process of a method according to another embodiment of the invention;
Fig. 7 shows a flow diagram of a learning process of a method according to another embodiment of the invention;
Fig. 8 shows a flow diagram of a synchronization of a method according to another embodiment of the invention;
Fig. 9 shows a flow diagram of a synchronization of a method according to another embodiment of the invention; and
Fig. 10 shows a template matching technique.
Fig. 11 shows a mapping.

DETAILED DESCRIPTION OF EMBODIMENTS

Throughout the figures, the same or corresponding features/elements of the various embodiments are designated with the same reference numbers.

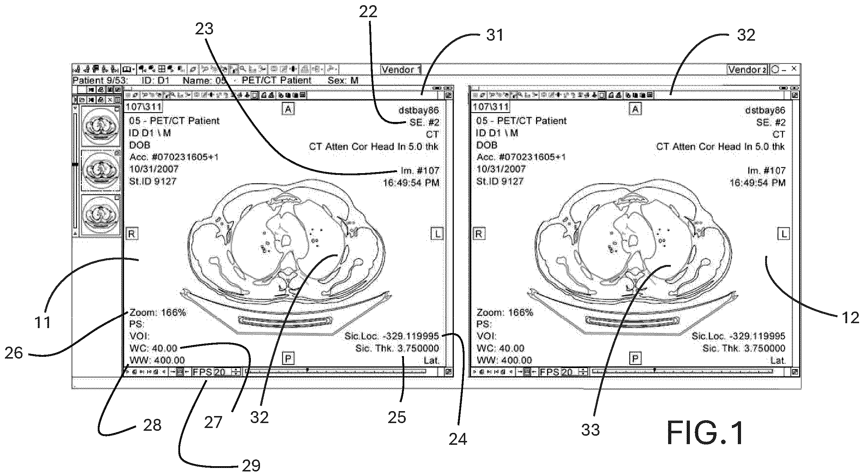

Fig. 1 shows an example of screen capture content of an image display showing a first processing application 11 simultaneously with a second processing application 12.

The first processing application 11 has access to image data or an image data set. The second processing application 12 has access to the same image data or image data set. The first processing application 11 is able to process and display an image from the image data. The second processing application 12 is able to process and display the image from the image data in a different manner compared to the processing of the first processing application 11.

A result of such processing is respectively displayed in a first window 31 and a second window 32 of the first processing application and the second processing application 12.

The word first in first window 31 denotes that it refers to a window of the first application 11. The word second in second window 32 denotes that it refers to a window of the second application 11.

The screen capture content displays medical image data 30 and multiple instances of window characteristic information that can be used with a method according to this invention.

Here, within the first window 31, first medical image data 33 is displayed. In the second window 32, second medial image data 34 is displayed.

The first medical image data 33 may be different from the second medical image data 34 because it may be processed differently. Typically, the first medical image data 33 is processed according to the first processing application 11 and the second medical image data 34 is processed according to the second processing application 12. Here, the first window 31 and the second window 32 have been synchronized in that the first medical image data 33 is synchronized with the second medical image data 34. For example, the first window 31 and the second window 32 have similar sizes and provide their respective medical image data in a similar view.

In this example, the window characteristic information comprises an accession number 21, a series number 22, an image number 23, a slice location 24, a slice thickness 25, a zoom factor 26, a window centre value 27, a window width value 28 and a slice number in a series 29.

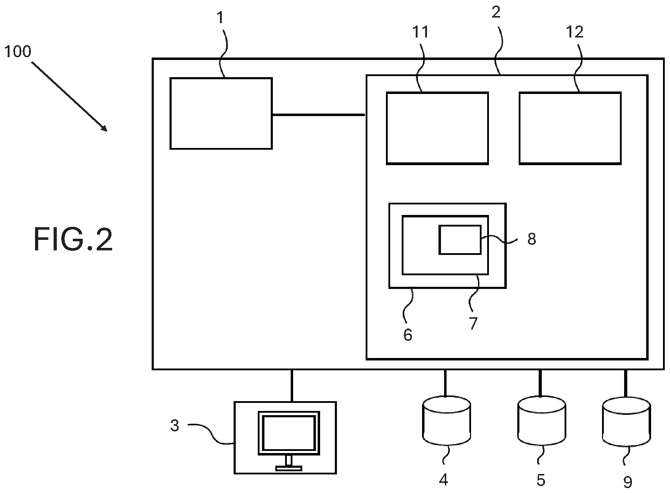

Fig. 2 shows a schematic describing an image visualisation and/or processing computing system 100 according to an embodiment of the invention.

The system 100 comprises a processing unit 1 and a computer-readable storage medium 2. On the storage medium 2 are stored a first processing application 11 and a second processing application 12. The first processing application 11 is configured to at least partially display image data in particular first medical image data 33. The second processing application 12 is configured to at least partially display the same image data but processed differently, in particular second medical image data 34.

For example, the image data may be loaded into the processing application 11, 12 subsequently processed and displayed on a computer screen 3 within the first window 31 and second window 32 respectively.

Preferably, the second processing application 12 is also configured to display image data, e.g. on the computer screen 3, simultaneously with the first processing application 11.

The first processing application 11 is configured to load the image data from a first data storage of image data 4. The second processing application 12 is configured to load image data from a second data storage of image data 5. The first data storage of image 4 and the second data storage of image data 5 may be part of the system or may be a remote data storage, e.g. a data server.

Optionally, the first data storage of image data 4 and the second data storage of image data 5 may be identical, i.e. the first processing application 11 and the second processing application 12 may optionally load image data from the same data storage 4, 5.

Both processing applications 11, 12 are typically configured to process image data to at least some extend. In particular, the second processing 12 application may have some processing or displaying functionality that the first processing application 11 does not provide. Hence, it may be desirable for a user, while working with the first processing application 11, to quickly switch to the second processing application 12 in order to access its additional functionality. It would be advantageous if the image data displayed in the first window 31 and the second window 32 are synchronized.

In order to provide an efficient workflow for this process, the system comprises a computer program 6 with a window characteristic recognition program 7 and instructions which, when the program is executed by the processing unit 1 causes the system to carry out a method according the invention.

Embodiments of this method are described in the following with respect to the figures. The window characteristic recognition program 7 has access to a library of screen locations 9 of window characteristic information and comprises a window characteristic recognition algorithm 8, such as an optical character recognition (OCR) algorithm.

Alternatively, the window characteristic recognition program 7 may have access to a suitable remote window characteristic recognition algorithm 8. For example, the window characteristic recognition program 7 may access an online window characteristic recognition algorithm 8. The window characteristic recognition algorithm 8 is configured to recognize window characteristic information at a particular screen location. Such window characteristic information is information suitable to identify the characteristics of a window in which image data is displayed.

In particular, the window characteristic recognition algorithm 8 may be configured to recognize window characteristic information in text form, such as letters or numbers.

The library 9 may be part of the system or may be a remote library. Optionally, the recognition program 7 may be further configured to recognize application identification information on screen capture content for identifying the first processing application 11. The application identification information may be stored in the library of screen locations 9 together with the screen locations.

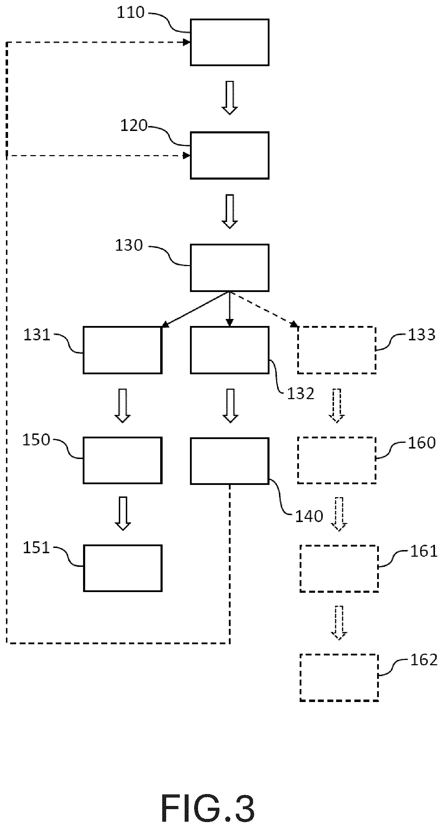

Fig. 3 shows a flow diagram of a computer-implemented method according to an embodiment of the invention. The method may be performed with a computing system as described with respect to Fig. 2 or with another suitable computing system. The method comprises the following steps: in a first step 110, a window characteristic recognition program 7 obtains screen capture content of at least part of an image display of a first processing application 11.

The image display is based on image data at least partially loaded into the first processing application 11. The screen capture content may in particular be a screenshot of at least part of an image display of the first processing application 11 that is currently reviewed by a user.

In a further step 120, a window characteristic recognition program 7 automatically attempts to retrieve window characteristic information from the screen capture content based on at least one screen location of window characteristic information from a library of screen locations 9 and by applying a window characteristic recognition algorithm 8.

In a further step 130 the screen capture content is classified. The screen capture content is classified as recognizable 131 if window characteristic information can be retrieved.

In an alternative, a further classification is performed to see, based on the retrieved window characteristic information, whether the image data can be identified in a data storage of image data 4,5 the second processing application 12 has access to. However, this is typically not necessary, because both processing applications 11, 12 have access to the same image data.

The screen capture content is classified as non-recognizable 132 if the library of screen locations 9 currently does not contain any screen locations and/or if no window characteristic information can be retrieved.

Optionally, the screen capture content may further be classified as non-findable 133 if window characteristic information can be retrieved and, based on the retrieved window characteristic information, no image data can be identified in a data storage of image data 4, 5 the second application has access to.

In a further step 140, which is applied in case the screen capture content is classified as non-recognizable, the window characteristic recognition program 7 performs a learning process to determine at least one screen location of window characteristic information on the image display based on changes happening over time on the image display and/or based on user input and storing the at least one screen location of window characteristic information in the library of screen locations 9.

Optionally, after storing the at least one screen location of window characteristic information in the library of screen locations 9 during the learning process, the method may be applied again starting with the step of obtaining screen capture content 110 or starting with the step of attempting to retrieve window characteristic information 120.

In particular, the learning process may thus be applied repeatedly to acquire more reliable screen locations and/or after the learning process has led to screen locations that are precise enough, the screen locations may be used for recognizing the window characteristic information when repeating the method.

In a further step 150, which is applied in case the screen capture content is classified as recognizable, the window characteristic information is provided to the second processing application 12 and the window 32 of the second processing application is synchronized with the window 31 of the first processing application based on the received window characteristic information of the first processing application.

An example of such synchronization is when the window characteristic information is a zoom factor 26. If the value of the zoom factor 26 is recognized (e.g. "166%" in Fig. 1), the same zoom factor may be applied to the second window 32 corresponding to the second processing application.

In another example of synchronization, the window characteristic information is a window centre value 27. If the window centre value 27 is recognized (e.g. "40.00" in Fig. 1), the same window centre value 27 may be applied to the second window 32 corresponding to the second processing application.

In another example of synchronization, the window characteristic information is a window width value 28. If the window width value 28 is recognized (e.g. "400.00" in Fig. 1), the same window centre value 28 may be applied to the second window 32 corresponding to the second processing application.

In another example of synchronization, the window characteristic information is an accession number 21. If the accession number 21 is recognized (e.g. "#070231605+1" in Fig. 1), the image data in the second window 32 corresponding to the second processing application is adapted such that it corresponds to the recognized accession number 21.

Similarly, in other examples of synchronization, the window characteristic information is one of a series number 22, an image number 23, a slice location 24 and/or a slice thickness 25. When these window characteristic information values are recognized, then second window 32 corresponding to the second processing application is adapted such that it corresponds to the respective series number 22, an image number 23, a slice location 24 and/or a slice thickness 25. Corresponding in this context means that the appearance and the image data displayed in the second processing applications relate to each other. So the image data displayed in the second processing application 12 relates to the same series number or the same image or the same slice location or has the same slice thickness.

After providing the window characteristic information to the second processing application 12, optionally, in a further step 151, based on the window characteristic information, the corresponding image data may at least partially be loaded from the data storage 4,5 into the second processing application 12 to synchronize the image data.

In a further optional step 161, which is applied in case the screen capture content is classified as non-findable, a user prompt is output notifying a user that the image data is missing and/or asking the user to verify the window characteristic information. If the optional steps 160, 161 are applied, optionally a further step 162 may be applied, wherein in case the screen capture content is classified as non-findable and/or in case a user input is received that verifies the window characteristic information, a further data storage 4 of image data the first processing application 11 is retrieving its image data from is accessed, and image data corresponding to the window characteristic information is located in the further data storage 4 and, if located, transferred to the data storage 5 the second application has access to and/or to the second processing application 12.

In an advantageous embodiment, the step of obtaining 110 also comprises a step of obtaining screen capture content of at least part of an image display of the second processing application 12. In other words, the obtained screen capture is from an image display of both the first and second processing applications 11, 12. In case the screen capture content from the first processing application 11 is classified as recognizable then the steps as described above applies. However, at the same time, in case the screen capture content from the second processing application 12 is classified as recognizable, then the method comprises the step of providing the window characteristic information to the first processing application 11 and synchronizing the first window 31 of the first processing application 11 based on the received window characteristic information of the second processing application 12.

Similarly, in case the screen capture content (based on the second processing application) is classified as non-recognizable, then the step of performing the learning process comprises the step of determining at least one screen location of window characteristic information on the image display of the second processing application 12 based on changes happening over time on the image display of the second processing application 12 and/or based on user input and storing the at least one screen location of window characteristic information in the library 9 of screen locations.

This way, synchronization may be two-way. In other words, it is possible to provide the window characteristic information from a screen capture in the first processing application 11 to the second processing application 12 and vice versa. It is then also possible to synchronize the second window 32 of the second processing application 12 based on the received window characteristic information of the first processing application 11 and vice versa, i.e. synchronize the first window 31 of the first processing application 11 based on the received window characteristic information of the second processing application 12.

This allows to show the same image data processed by two different image processing applications 11, 12 in a synchronized manner.

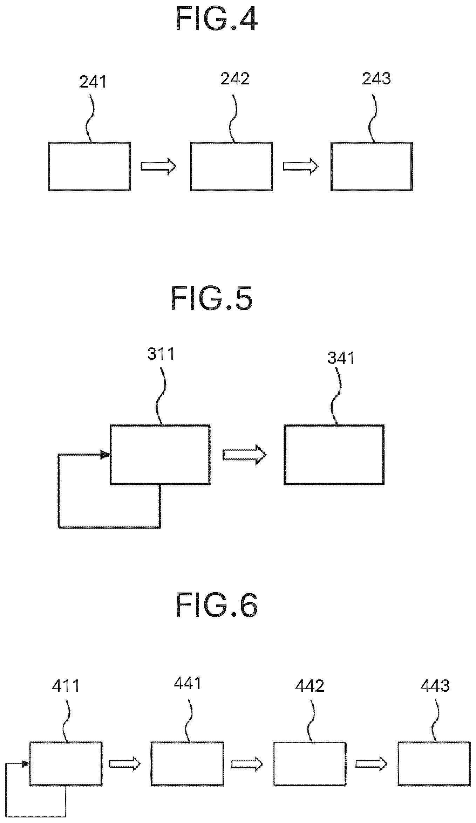

Fig. 4 shows a flow diagram of a learning process of a method according to an embodiment of the invention. In particular, the step of performing a learning process according to this embodiment may be applied in step 140 of the method described with respect to Fig. 3.

According to this embodiment, the learning process comprises a step 241 of tracking a user input concerning a selection of image data. In a further step 242 windows characteristic information is retrieved from the selected image data, for example from the metadata of the image data. In a further step 243, the window characteristic information is searched on the screen capture content via the window characteristic recognition algorithm 8 and, thus, the screen location of the corresponding window characteristic information is located on the screen capture content.

Fig. 5 shows a flow diagram of a learning process of a method according to another embodiment of the invention. In particular, the step of performing a learning process according to this embodiment may be applied in step 140 of the method described with respect to Fig. 3. First, a step of obtaining screen capture content 311 is applied multiple times and/or continuously. This may be done from screen capture content corresponding to the first processing application 11 and/or from screen capture content corresponding to the second processing application 12. For example, the screen capture content as displayed in Fig. 1 may be used.

Further, in the step 341 of the learning process one or multiple sections of the screen capture content that are changing over time are analysed and it is determined that or whether the at least one screen location of window characteristic information is within a changing section of the screen capture content.

For example, text recognized within the changing sections may be analysed and it may be determined whether the text is or comprises window characteristic information. For example, text recognized within the changing section may be analysed whether it fits a type of window characteristic information (e.g. having the same number of digits and/or a same format etc.) and/or whether this window characteristic information can be found in the metadata of a database of image data.

Fig. 6 shows a flow diagram of a learning process of a method according to another embodiment of the invention. In particular, the step of performing a learning process according to this embodiment may be applied in step 140 of the method described with respect to Fig. 3. First, a step of obtaining screen capture content 411 is applied multiple times and/or continuously. In a further step 441 a user input concerning a selection of image data or change in a user interface is tracked. In a further step 442 window characteristic information is retrieved from the selected image data or displayed user interface, for example from the metadata of the image data or what can be seen on the image display. In a further step 443, the window characteristic information is searched in the one or multiple changing sections of the screen capture content via the window characteristic recognition algorithm 8 and, thus, the screen location of the corresponding window characteristic information is located on the screen capture content.

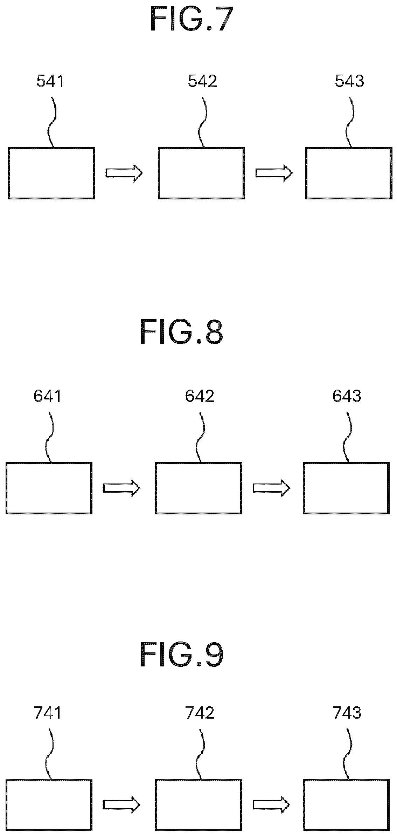

Fig. 7 shows a flow diagram of a learning process of a method according to another embodiment of the invention. In particular, the step of performing a learning process according to this embodiment may be applied in step 140 of the method described with respect to Fig. 3. This embodiment may be used as an alternative to the embodiments shown in Figs. 4-6. This embodiment may be combined with the embodiments shown in Figs. 4-6. For example, this embodiment and the embodiment described with respect to Fig. 4 may be combined in a similar manner as the embodiments of Fig. 4 and Fig. 5 are combined in the embodiment of Fig. 6. In one step 541, the window characteristic recognition program 7 provides a user interface that allows a user the option to select window characteristic information on the image display of the first processing application 11 to input at least one window characteristic information selection. In a further step 542, the window characteristic information selection is received by the window characteristic recognition program 7. In a further step 543, based on the window characteristic information selection, the at least one screen location of window characteristic information is determined by the window characteristic recognition program 7.

Fig. 8 shows a flow diagram of a step of synchronization according to another embodiment of the invention. In particular, the step of synchronization according to this embodiment may be applied in step 150 of the method described with respect to Fig. 3.

In this embodiment, in a step 641 the window characteristic information is provided to the second processing application 12. In a further step 643, the window of the second processing application is synchronized and comprises the step of synchronizing by adjusting the zoom factor and/or panning settings of the image data within the second window resulting in a similar field of view for the first window 31 based on the received window characteristic information.

In other words, the second window 32 of the second processing application 12 is adjusted according to the recognized and identified window characteristic information of the first window 31.

In an example, the window characteristic information is a displayed image title. The zoom factor is often present in a displayed image title. In other words, the first processing application 11 may display a zoom factor in the displayed image title in an explicit or implicit way. It may be possible to derive the zoom factor from the image title. In other words, the image title of the first processing application 11 is used to display the image data in the second window 32 of the second processing application 12 with the same zoom.

Alternatively, the zoom factor may be derived directly from the image display of the first processing application as described earlier.

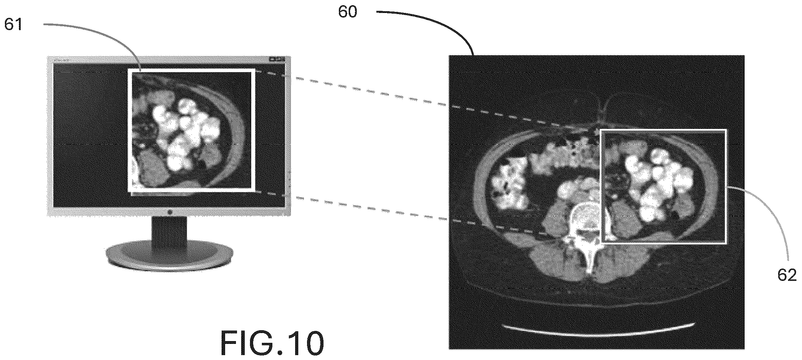

Optionally, for example when the zoom factor or panning settings are not available, a step 642 of applying a template matching technique may be used. The displayed original image 61 in the first processing application 11, when zoomed/panned, may be a small part image portion 62 of an original full image 60 and thus can be used as a template. This is illustrated in Fig. 10. Since the second processing application 12 has access to the original full image 60, it can use template matching with the displayed image in the first processing application 11 to find what small part image portion 62 of the original full image 60 is displayed and calculate the zoom and or pan values from it.

This is typically advantageous to identify an image shift (pan) in the first processing application to apply to/synchronize the image data in the second processing application 12.

Fig. 9 shows a flow diagram of a step of synchronization according to another embodiment of the invention. In particular, the step of synchronization according to this embodiment may be applied in step 150 of the method described with respect to Fig. 3. However, it may be combined with any previous disclosed synchronization technique.

Again, in a step 741 the window characteristic information is provided to the second processing application 12.

In a further step 743, synchronizing the window of the second processing application is synchronized by windowing the window, resulting in a similar window of a grey-level mapping.

Typically, window centre and window width values are present on a displayed image which was illustrated in Fig. 1 by means of the window centre value 27 and window width value 28.

A preferred embodiment is to synchronize the second window based on the recognized and identified window centre value 27 and/or window width value 28 as previously described.

In an optional embodiment, to identify the location of the window centre value 27 and the window width value 28 on the screen an initial calibration procedure is applied, where the user is changing the first window 31 manually. The second processing application 2 recognizes the changing area on the screen using character recognition and saves this location for future identification of the window centre value 27 and/or window width value 28.

If the window centre value 27 and/or window width value 28 are not available for identification from the screen, a different method may be used.

Preferably, the zoom factor and/or panning settings have been identified according to a previous described embodiment in an optional but preferred step 742.

Windowing according to this embodiment is a mapping of higher resolution image data into lower resolution images for display, wherein the image before mapping has a higher resolution compared to the image for display after mapping. The higher resolution image data corresponds to a real-world original image data and the lower resolution image data corresponds to stored displayed image data.

In particular, windowing is a mapping of 12-bit CT/MR images into 8-bit grey-scale images for display.

In the first processing application 11 an 8-bit grey-scale image is available for displaying. The second processing application has access to the original same 12-bit image used in the first processing application 11.

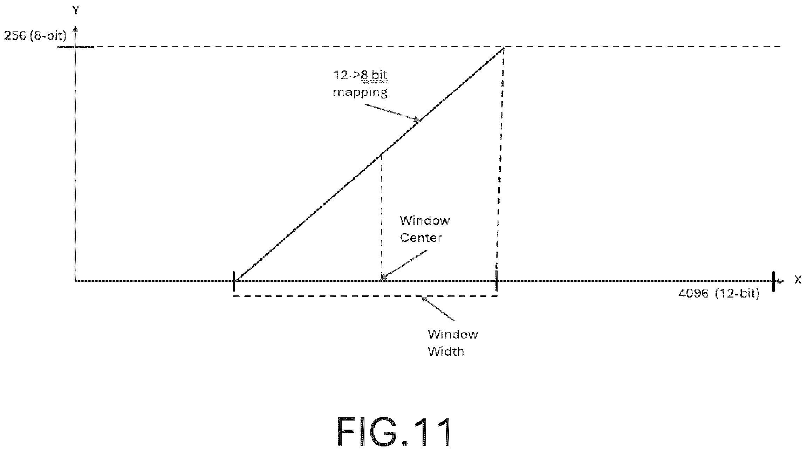

Based on this, mapping values for the window centre value and/or the window width value may be determined, which is further illustrated in Fig. 11.

A linear equation of the mapping (reverse, from Y to X) can be presented as:

$$X = a * Y + b$$

With reference to Fig. 11, X represents the higher resolution image (4096, 12 bit) and Y represents the lower resolution image (256, 8 bit).

We can seek for two different pixels from two different locations (x,y) on the image and get:

$$x1 = a * y1 + b$$

$$x2 = a * y2 + b$$

Here, x and y represent the location coordinates of said pixels.

From this we can calculate a and b:

$$a = (x2 - x1) / (y2 - y1)$$

$$b = x1 - a * y1$$

Finally, from there we can calculate a window centre value and a window width value:

$$Window\ centre\ value = a * 128 + b$$

$$\text{Window width value} = a * 256$$

The calculated window centre value and window width value may subsequently be used to synchronize the second window 32 of the second processing algorithm.

As an alternative option, instead of individual pixels, an average of several adjacent pixels, within a small rectangular (e.g., 2x2 or 3x3 pixels) may be used. This may increase accuracy.

The above discussion is intended to be merely illustrative of the present method, system and computer program and should not be construed as limiting the appended claims to any particular embodiment or group of embodiments. Accordingly, the specification and drawings are to be regarded in an illustrative manner and are not intended to limit the scope of the appended claims. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "an" or "a" does not exclude a plurality. A single processor or other unit may fulfil the functions of several items recited in the claims. The mere fact that certain measures are recited in mutually different dependent claims does not indicate that a combination of these measured cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS

1    processing unit
2    computer-readable storage medium
3    computer screen
4    first data storage of image data
5    second data storage of image data
6    computer program
7    window characteristic recognition program
8    window characteristic recognition algorithm
9    library
11   first processing application
12   second processing application
21   accession number
22   series number
23   image number
24   slice location
25   slice thickness
26   zoom factor
27   window centre value
28   window width value
29   slice number in a series
31   first window
32   second window
33   first medical image data
34   second medical image data
100  image visualisation and/or processing computing system

**Claims**

1.  A computer-implemented method of image viewing and/or processing within an image viewing and/or processing environment, the environment comprising at least a first processing application (11) being configured to receive, process and display image data (33, 34) in a window (31, 32), a second processing application (12) being configured to receive, process and display image data in a window, a window characteristic recognition program (7) having access to a library (9) of screen locations of window characteristic information and comprising or having access to a window characteristic recognition algorithm (9) that is configured to recognize window characteristic information at a particular screen location, wherein the window characteristic information is information suitable to identify the characteristic of a window in which image data is displayed, the method comprising the following steps:

    - obtaining, by the window characteristic recognition program, screen capture content of at least part of an image display of the first processing application, wherein the image display is based on image data at least partially loaded into the first processing application;
    - automatically attempting to retrieve window characteristic information, by the window characteristic recognition program, from the screen capture content based on at least one screen location of window characteristic information from the library of screen locations;
    - classifying the screen capture content, by applying the window characteristic recognition algorithm:

        - as recognizable if window characteristic information can be retrieved;
        - as non-recognizable if the library of screen locations currently does not contain any screen locations and/or if no window characteristic information can be retrieved;

        in case the screen capture content is classified as recognizable:

- providing the window characteristic information to the second processing application;
- synchronizing the window of the second processing application based on the received window characteristic information of the first processing application;

in case the screen capture content is classified as non-recognizable:

- performing a learning process, by the window characteristic recognition program, to determine at least one screen location of window characteristic information on the image display based on changes happening over time on the image display and/or based on user input and storing the at least one screen location of window characteristic information in the library of screen locations.

2. The method according to any of the preceding claims, wherein the step of synchronizing the window of the second processing application comprises the step of:

- synchronizing by adjusting a zoom factor and/or panning setting for the image data (33, 34) within the window (31, 32) resulting in a similar field of view for the window of the first processing application (11) and the window of the second processing application (12).

3. The method according to any of the preceding claims, wherein the step of synchronizing the window of the second processing application comprises the step of:

- synchronizing by windowing the window (31, 32), resulting in a similar window of a grey-level mapping.

4. The method according to any of the preceding claims, wherein the method further comprises the step of:

- displaying the window (31) of the first processing application and the window (32) of the second processing application simultaneously.

5. The method according to any of the preceding claims, wherein the step of performing the learning process is performed while the first processing application is running.

6. The method according to any of the preceding claims, wherein the step of performing the learning process further comprises:

- tracking changes on the image display that are due to user input and/or registering user input.

7. The method according to any one of the preceding claims, wherein the step of performing the learning process comprises:

- tracking a user input concerning a selection of image data (33, 34);
- retrieving window characteristic information from the selected image data; and
- searching for the window characteristic information on the screen capture content via the window characteristic recognition algorithm (8) to locate the screen location of the corresponding window characteristic information on the screen capture content.

8. The method according to any one of the preceding claims, wherein the step of obtaining screen capture content comprises obtaining the screen capture content multiple times and/or continuously and wherein the step of performing the learning process comprises analysing one or multiple sections of the screen capture content that are changing over time and determining that or whether the at least one screen location of window characteristic information is within a changing section of the screen capture content.

9. The method according to any of the preceding claims, wherein the step of performing the learning process comprises searching for the window characteristic information via the window characteristic recognition algorithm (8) in the one or multiple changing sections of the screen capture content.

10. The method according to any one of the preceding claims, wherein the step of performing the learning process comprises:

- providing a user interface that allows a user an option to select window characteristic information on the image display of the first processing application (11) to input at least one window characteristic information selection;
- receiving the window characteristic information selection by the window characteristic recognition program (7); and
- determining by the window characteristic recognition program and based on the window characteristic information selection, the at least one screen location of window characteristic information.

**11.** The method according to any one of the preceding claims,

wherein the window characteristic information comprises at least one or more of the following:
- an image number (23), an image location, an image description, a slice number, a slice location (24), a slice description, a slice thickness (25), a series number (22), a series description, an accession number (21), a zoom factor (26), a window centre value (27), a window width value (28), a slice number in a series (29); and/or
- wherein the image data comprises medical image data, in particular comprising at least one or more of the following: computed tomography image data, photon counting image data, spectral computed tomography image data, magnetic resonance image data, nuclear imaging data, positron emission tomography data, single photon emission computed tomography imaging data, ultrasound image data, digital pathology data and/or digital X-ray radiogrammetry data.

**12.** The method according to any one of the preceding claims, wherein the method comprises the steps of:

- storing the at least one screen location of window characteristic information in the library (9) of screen locations during the step of performing the learning process;
- applying again the previous steps starting with the step of obtaining screen capture content or starting with the step of attempting to retrieve window characteristic information.

**13.** A computer program (6) comprising instructions which, when the program is executed by a computer, cause the computer to carry out the method according to any one of the preceding claims.

**14.** An image visualisation and/or processing computing system (100) comprising a processing unit (1) adapted to perform the steps of the method according to any of the claims 1-12.

**15.** The image visualization and/or processing computing system according to claim 14, comprising a data storage (4, 5) that comprises a library (9) of screen locations.

FIG.1

FIG.2

100

1

11

2

12

6

7

8

4

5

9

3

# FIG.3

# FIG.4

# FIG.5

# FIG.6

# FIG.7

541　　　　　　　　542　　　　　　　　543

```
┌─────┐      ┌─────┐      ┌─────┐
│     │  ⇒   │     │  ⇒   │     │
└─────┘      └─────┘      └─────┘
```

# FIG.8

641　　　　　　　　642　　　　　　　　643

```
┌─────┐      ┌─────┐      ┌─────┐
│     │  ⇒   │     │  ⇒   │     │
└─────┘      └─────┘      └─────┘
```

# FIG.9

741　　　　　　　　742　　　　　　　　743

```
┌─────┐      ┌─────┐      ┌─────┐
│     │  ⇒   │     │  ⇒   │     │
└─────┘      └─────┘      └─────┘
```

FIG.10

256 (8-bit)

Y

12->8 bit
mapping

Window
Center

Window
Width

4096 (12-bit)

X

FIG.11

Europäisches
Patentamt

European
Patent Office

Office européen
des brevets

# EUROPEAN SEARCH REPORT

**Application Number**

EP 24 21 0148

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/062956 A1 (GOTMAN SHLOMO [IL] ET AL) 3 March 2016 (2016-03-03) * paragraph [0001] - paragraph [0068]; claims 1-20; figures 1-10 * ----- | 1-15 | INV. G06V10/22 G06V10/25 G06V10/764 G06V10/94 G06V30/14 G06V30/19 G06F3/048 G16H30/20 G16H30/40 |

**TECHNICAL FIELDS SEARCHED (IPC)**

G06V
G06F
G16H

The present search report has been drawn up for all claims

| Place of search | Date of completion of the search | Examiner |
|---|---|---|
| The Hague | 12 April 2025 | Fredj, Aziz |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 738 273 A1

## ANNEX TO THE EUROPEAN SEARCH REPORT
## ON EUROPEAN PATENT APPLICATION NO.

EP 24 21 0148

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2025

| Patent document cited in search report | Publication date | Patent family member(s) | | Publication date |
|---|---|---|---|---|
| US 2016062956 A1 | 03-03-2016 | CN | 105144175 A | 09-12-2015 |
| | | CN | 113096777 A | 09-07-2021 |
| | | EP | 2989575 A2 | 02-03-2016 |
| | | JP | 6433983 B2 | 05-12-2018 |
| | | JP | 2016522705 A | 04-08-2016 |
| | | US | 2016062956 A1 | 03-03-2016 |
| | | WO | 2014174461 A2 | 30-10-2014 |

EPO FORM P0459

For more details about this annex : see Official Journal of the European Patent Office, No. 12/82

25